# EUROPEAN PATENT SPECIFICATION

(11) **EP 1 581 116 B1**
(45) Date of publication and mention of the grant of the patent: **07.04.2010**
(21) Application number: 03814858.1
(22) Date of filing: 17.12.2003
(51) Int. Cl.: A61B 5/15

(54) **CAPILARY TUBE TIP DESIGN TO ASSIST BLOOD FLOW**
KAPILLARROHR-SPITZEN-DESIGN ZUR UNTERSTÜTZUNG DES BLUTFLUSSES
CONFIGURATION DE POINTE DE TUBE CAPILLAIRE DESTINEE A ASSISTER LE FLUX SANGUIN

(30) Priority: 30.12.2002 US 437081 P
(43) Date of publication of application: 05.10.2005
(73) Proprietor: Roche Diagnostics GmbH, 68298 Mannheim (DE); F. Hoffmann-La Roche AG, 4070 Basel (CH)
(72) Inventor: ROE, Jeffrey, N., San Ramon, CA 94583 (US); ROE, Steven, N., San Mateo, CA 94403 (US); RANEY, Charles, C., Camdenton, MO 65020 (US)
(86) International application number: PCT/US2003/040330
(87) International publication number: WO 2004/060163

(56) References cited:
- WO-A-02/056751
- US-A- 4 360 016
- US-A- 4 441 373
- US-B1- 6 183 489

## Description

### BACKGROUND

The present invention relates generally to lancing devices and methods for obtaining body fluid samples from the body for analysis, and more specifically, but not exclusively, to capillary tubes used in the above mentioned devices.

Many medical procedures in use today require a relatively small sample of blood, in the range of 3-50 milliliters. It is more cost effective and less traumatic to the patient to obtain such a sample by lancing or piercing the skin at a selected location, such as the finger, to enable the collection of 1 or 2 drops of blood, than by using a phlebotomist to draw a tube of venous blood. With the advent of home use tests such as self-monitoring of blood glucose, there is a requirement for a simple procedure, which can be performed in any setting by a person needing to test.

In institutional settings, it is often desirable to collect the sample from the patient and then introduce the sample to a test device in a controlled fashion. Some blood glucose monitoring systems, for example, require that the blood sample be applied to a test device that is in contact with a test instrument. In such situations, bringing the finger of a patient directly to the test device poses some risk of contamination from blood of a previous patient. With such systems, particularly in hospital settings, it is common to lance a patient, collect a sample in a micropipette via capillary action and then deliver the sample from the pipette to the test device.

Conventional methods and devices for sampling blood or other bodily fluids require piercing the skin with a sharp object like a lancet to from an incision in the skin and underlying blood vessels and subsequently obtaining the sample with a capillary tube. An end of the capillary tube is inserted into the drop of blood that formed on the surface of the skin at the incision. The blood enters the capillary tube by capillary action. The capillary tube is then used to transfer the blood to a test device or collection device.

A blood sample is commonly taken from the fingertips, where the blood supply is generally excellent. However, because some patients must perform multiple tests daily, the fingertips may become sensitive or calloused thereby making it difficult to obtain a sample from the fingertips. Additionally, the nerve density in the fingertip region is greater than in other areas of the body. As a result, patients may experience pain or discomfort when the incision is made. To avoid the pain and discomfort, alternate sampling points, such as earlobes and limbs, are sometimes used for obtaining a bodily fluid sample. The disadvantage to the alternate sampling points is that the blood capillary density is also lower, thus reducing the size of the sample.

Using these alternative sampling points often requires a patient, who is obtaining a self-sample, to perform the procedure using only one hand to create the incision and to obtain the sample. Alternatively, the patient may have to look into a mirror in order to see the sampling point. In either situation, the patient may have difficulty performing the procedure because of the high degree of coordination involved. In particular, the patient must lance the skin using a lancing device, put the lancing device down, then, using the same hand in which the lancing device was held, pick up the capillary tube and insert the end of the capillary tube into the blood drop that had formed on the surface of the skin at the incision. A possibility exists, during this procedure, that the blood may drip from the incision onto the floor, other clothing, or even another person. The blood sample itself may also become contaminated. Additionally, the blood may be accidentally smeared over the patient's skin and have to be cleaned up causing the cleanup items to be contaminated with blood.

Another drawback to the procedure described above is it requires the use of two separate devices, a lancing device for creating the incision in the skin, and a capillary tube for obtaining the sample. The requirement for multiple devices is likely to lead to difficulty in performing the procedure due to misplacing or forgetting one of the two devices. Moreover because it requires multiple devices and multiple steps, this procedure is too time consuming.

One approach for eliminating the problems inherent with using two separate devices is to combine the two separate devices into one device. While this approach does reduce the number of devices, the device must be removed from the sample location after making the incision, and mechanically adjusted to switch from a lancing device to a capillary tube device. Thus, while this approach reduces the number of devices, the patient must still have a degree of coordination and may experience the clean up and contamination problems discussed above.

Additionally, a patient may also have difficulty obtaining the desired sample size because of the capillary tube itself. If the patient presses the capillary tube into the skin around the incision, the compression of the wound may stop the blood flow out of the incision. The patient would then likely struggle with the wound in order to restore the blood flow to obtain a larger sample, or the patient may repeat the sampling procedure at a different sampling point.

The document US 4,360,016 describes a lancing device in which a capillary tube is positioned next to a lancet. The lancet and the tube are within a housing of the device which has a front end to contact the skin. The front end of the housing is inclined so that the tube is not directly pressed against the finger and blood can flow out of the wound into the capillary. Furthermore document WO 02/056751 reveals a system having a lancet within a capillary tube. The end of the tube is pressed against the finger to drawn up bodily fluid.

For the foregoing reasons, there is a need for a fluid sampling device and method that can create an incision and obtain the bodily fluid sample without being removed from the sample location and operable using only one hand. Additionally, there is a need for a fluid sampling device with a capillary tube that does not inhibit the flow of blood out of the incision. Moreover, there is a need for such a device to be disposable.

### SUMMARY

One form of the present invention concerns a body fluid sampling device that includes an incision forming member adapted to form an incision in the skin, a capillary member with a capillary tube tip to maintain the incision open when the body fluid sampling device is pressed into the skin. The incision forming member travels longitudinally within the capillary member.

In another embodiment, the capillary tube tip applies an asymmetric force around the incision to maintain the incision open.

In yet another embodiment the fluid sampling device has grooves defined in the side of the capillary member which allow air to flow into the capillary member where the capillary member contacts the skin to facilitate the blood flow into the capillary member.

In an additional embodiment the fluid sampling device is disposable.

In another embodiment the capillary tube tip is flared outwardly.

Another form is directed to a method of obtaining a body fluid sample through an incision in the skin where a capillary tube tip is pressed into the skin at a sample point. An incision is created by piercing the skin with an incision forming member. The incision forming member is retracted from the incision. The capillary tube tip asserts an outward force around the incision in such a way that the incision remains open. Body fluid travels into the fluid receiving space. The capillary tube tip is pulled away from the skin.

In an additional embodiment, the fluid receiving space includes a testing element for testing the body fluid while the body fluid is in the fluid receiving space.

Further forms, objects, features, aspects, benefits, advantages, and embodiments of the present invention will become apparent from a detailed description and drawings provided herewith.

### BRIEF DESCRIPTION OF THE DRAWINGS

FIG.1 is a cross-sectional view of a fluid sampling device according to one embodiment.
FIG. 2 is a cross-sectional view of a fluid sampling device according to another embodiment.
FIG. 3 is a perspective view of a fluid sampling device according to an additional embodiment.
FIG. 4 is a perspective view of a fluid sampling device according to a further embodiment.
FIG. 5 is a perspective view of a fluid sampling device according to another embodiment.
FIG. 6 is a perspective view of a fluid sampling device according to yet another embodiment.
FIG. 7 is a cross-sectional view of the FIG. 1 fluid sampling device configured to lance at a skin site.
FIG. 8 is a cross-sectional view of the FIG. 1 fluid sampling device lancing the skin site.
FIG. 9 is a cross-sectional view of the FIG. 1 fluid sampling device after lancing the skin site.
FIG. 10 is a cross-section view of the FIG. 1 fluid sampling device where the incision forming member is in its extended position.

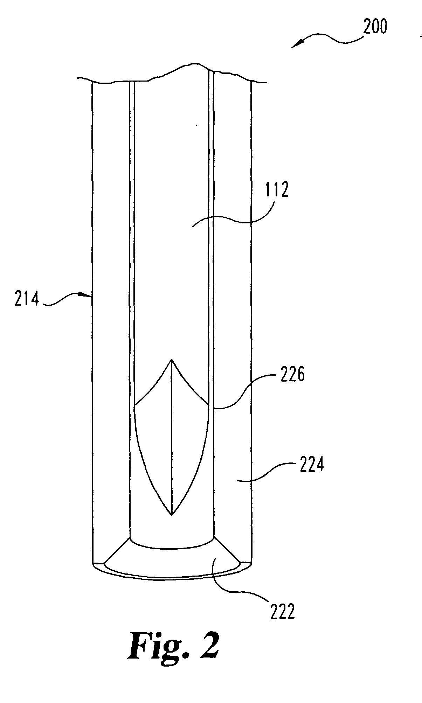

The embodiments of Figures 2-6 do not from part of the invention.

### DESCRIPTION OF SELECTED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates. One embodiment of the invention is shown in great detail, although it will be apparent to those skilled in the art that some of the features which are not relevant to the invention may not be shown for the sake of clarity.

The present invention is directed to a device that satisfies a need for a one-hand operable bodily fluid sampling device that can create an incision in the skin and subsequently obtain a sample of the bodily fluid in a capillary tube while remaining in contact with the patient's skin at the sampling location and maintaining the blood flow out of the incision.

The present invention locates the lancet within the capillary tube such that the capillary tube is positioned and centered over the incision before the incision lancet is extended to create the incision. This avoids the need for moving the capillary tube after the incision is made, and consequently reduces the significant difficulties that can be encountered in moving a capillary tube quickly and accurately to the site of an incision. Therefore, it enhances the ability to acquire the expressed body fluid without loss, delay, or contamination. Additionally, the tip of the capillary tube in contact with the skin is configured to spread the skin apart at the incision location to facilitate the flow of blood outwardly through the incision. In one configuration, the capillary tube tip is shaped asymmetrically to apply an asymmetric force around the incision to maintain the incision open. Moreover, the capillary tube tip in another embodiment has grooves in its side allowing air to flow into the capillary tube to facilitate the flow of blood into the capillary tube.

The present invention is useful with various body fluids. For example, the unit is suitable for accessing either blood or interstitial body fluid. The devices may be readily configured for either fluid by controlling the distance by which the lancing member extends into the user's skin when in the extended position. For example, a depth of 0.5 to 2.5 mm will typically produce blood.

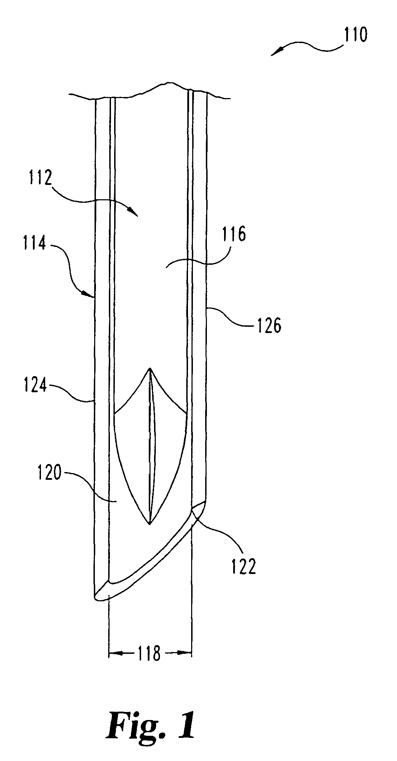

A bodily fluid sampling device 110 according to one embodiment of the present invention is illustrated in FIG. 1. The sampling device 110 includes an incision forming member 112 and a sample acquisition element 114. For the sake of clarity and brevity, other components of the sampling device that are well known in the art, such as springs, testing mechanisms and methods, hammers, cocking mechanisms, and the like that are not important to appreciate the present invention, will not be discussed below. For examples of such components please refer to U.S. Patent Application Serial No. 10/054,270 (Publication No. US 2002/0103499 A1).

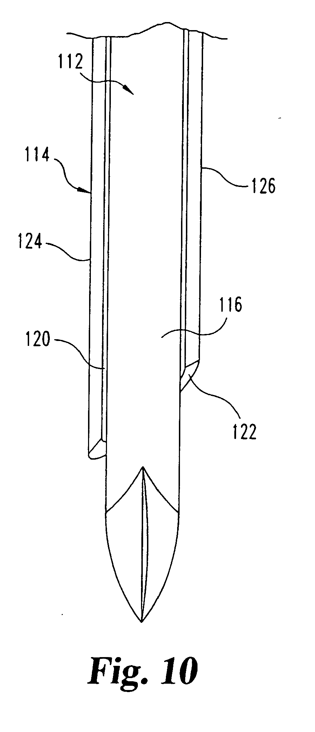

As shown in FIG. 1, the end of the incision forming member 112 includes a lancet 116 in the form of a needle. However, the lancet 116 can be in other forms, such as a blade. Additionally, although only a single lancet 116 is shown, the incision forming member 112 in other embodiments can include multiple lancets 116. The lancet 116 is movable longitudinally between a first, retracted position (FIG. 1), and a second, extended position (FIG. 10) within the sample acquisition element 114. The sample acquisition element 114 has an internal diameter 118 sized to draw and retain fluid from a contacted source using capillary action. In one embodiment, the sample acquisition element 124 is in the form of a capillary tube For example, the sample acquisition element 114 could be any device or material capable of receiving, holding, and/or transferring the body fluid to a testing device, such as a wricking material or a lateral transfer membrane. The sample acquisition element 114 may be made from any suitable material, and typically can be economically produced from plastics, glass, or various other materials, for example by injection molding or extrusion. Additionally, the sample acquisition element 114 may be manufactured of polyvinyl chloride or any similar biocompatible plastic.

A fluid receiving space 120 is defined within the sample acquisition element 114 and disposed between the incision forming member 112 and the internal wall of the sample acquisition element 114. Although in the illustrated embodiment the fluid receiving space 120 is cylindrical, the shape of the space will vary depending on the shapes of the lancet 116 and the sample acquisition element 114, and the position of the incision forming member 112 within the sample acquisition element 114. Preferably, the sample acquisition element 114 and the incision forming member 112 are sized such that the fluid receiving space 120 will hold the desired amount of fluid. Alternatively, the sample acquisition element 114 may be sized such that the interior volume of the sample acquisition element 114 will hold the desired amount of fluid when the incision forming member 112 is removed from the fluid sampling device 110.

The lancet 116 is received and longitudinally movable within the fluid receiving space 120 of the sample acquisition element 114 between a first, retracted position (FIG. 1), and a second, extended position (FIG. 10). Means, such as a spring, are provided for resiliently extending and retracting the lancing member in order to make a desired incision and to then withdraw the lancet back into a shielded position. Examples of such mechanisms are contained in the following U.S. Patent Nos, 5,951,492; 5,857,983; and 5,964,718. A resilient means is mounted to provide relative movement to retract the lancet into the main body after making the incision, Preferably the resilient means, such as a spring, is made from a biocompatible material, such as metal, plastic, elastic, or a similar material known in the art, which does not react with the sample or interfere with the testing procedure. The resilient means may allow multiple uses if the unit is to be reused, or may be a disposable or one-use mechanism used with disposable or one-use embodiments of the unit.

These devices typically extend the lancet to a defined extent, such as by moving the lancet to a stop. Such devices frequently are produced with a predefined limit of travel for the lancet, thereby defining a penetration for the lancet into the skin. Alternatively, these devices permit the user to adjust the penetration depth, such as by turning a wheel or other mechanism, with such adjustable devices frequently including a dial or other display which indicates the selected depth. These types of mechanisms are useful in combination with the present invention. Various means may similarly be employed for retracting the lancet 116 after it has made the incision, and many such mechanisms are known in the art, including the references previously cited.

The lancet 116 may be manufactured of any biocompatible material such as steel, surgical stainless steel, aluminum, or titanium, as well as many other suitable materials known in the art. Preferably, lancet 116 is made in a solid piece that is sufficiently sharpened to create an incision.

The withdrawal of the lancet 116 may also be either a full or partial withdrawal. When fully withdrawn, the lancet 116 is removed from the incision and returned to the retracted position (FIG. 1) protected from accidental contact by the user. However, in an alternate approach, the lancet 116 could be only partially withdrawn, thereby leaving a portion of the lancet 116 remaining within the incision. When the lancet 116 is only partially withdrawn, the lancet 116 acts as a focal point for locating the blood and transferring it to the sample acquisition element 114. Furthermore, in another approach the incision forming member 112 may be physically removed from the sampling device 110.

The sample acquisition element 114 has a skin contact surface 122 with an asymmetric slope. Referring to FIG. 1, a first side 124 of the sample acquisition element 114 is longer than the its opposing, second side 126. Due to this shape, the skin contact surface 122 is sloped between the first side 124 and the second side 126. The skin contact surface 122 applies an asymmetric force to the skin surrounding the incision prevent the skin on opposite sides of the incision from contacting each other, thus maintaining the incision open.

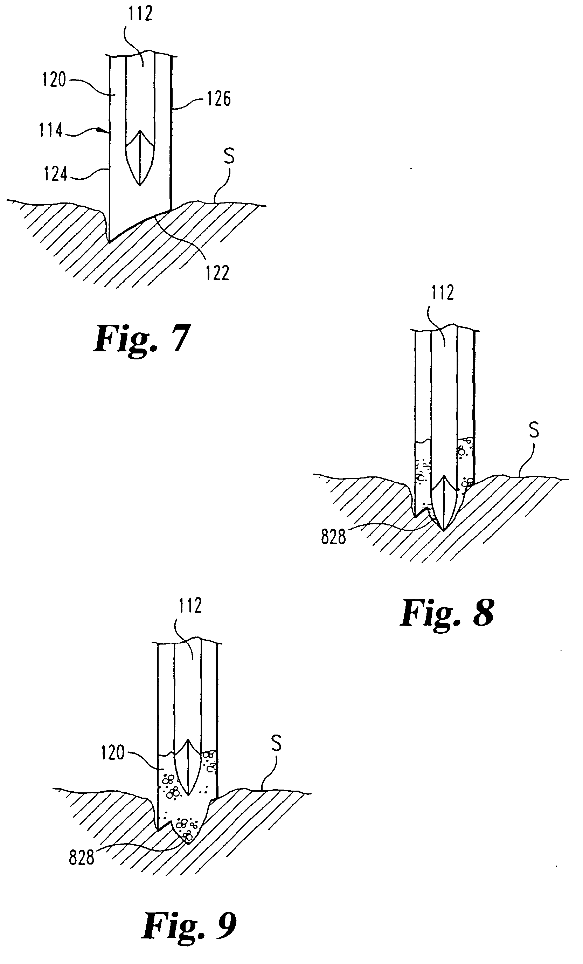

A technique for collecting a fluid sample with device 110 will now be described with reference to FIGS. 7-9. Initially, the skin contacting surface 122 is placed against the skin, as is shown in FIG. 7. When the sample acquisition element 114 is pushed perpendicularly into the skin at the sample location, the skin under the longer first side 124 deflects inwardly into the skin more than the skin under the shorter second side 126. As depicted in FIG. 7, the inward deflection under the skin contact surface 122 stretches the skin S within the sample acquisition element 114. During lancing, as is shown in FIG. 8, the incision forming member 112 is fired by a firing mechanism to form an incision 828 in the skin S. Afterward, as illustrated in FIG. 9, the incision forming member 112 is retracted into the sample acquisition element 114. The longer first side 124 maintains an outward force on the incision 828, preventing the skin S on opposite sides of the incision 828 from touching and closing the incision 828. Without having to move or remove the sample acquisition element 114, bodily fluid is drawn into the fluid receiving space 120 via capillary action. In another embodiment, the bodily fluid is deposited on a test strip positioned within the sample acquisition element 114. The test strip can then be used to analyze the fluid sample. Although the incision forming member 112 is illustrated as being positioned inside the acquisition element 114 during fluid collection, the incision forming member 112 in other embodiments, may be removed from the acquisition element 114.

A fluid sampling device 200 according to another embodiment will now be described with reference to FIG. 2. Device 200 shares a number of features similar to the one described above with reference to FIG. 1. For instance, device 200 includes an incision forming member 112, a sample acquisition element 214, and a skin contact member 226, However, in the embodiment illustrated in FIG. 2, the sample acquisition element 214 is a tube with a beveled opening. In particular, the sample acquisition element 214, of the illustrated embodiment, has a longer outside surface 224 than its inside wall 226, forming the skin contact surface 222 that is sloped between the longer outside wall 224 and the shorter inside wall 226.

The incision forming member 112 is movable longitudinally within the sample acquisition element 214 and operates in a manner similar to the one discussed above with reference to FIGS. 7-9. The incision forming member 112 is movable longitudinally between a first, retracted position, and a second, extended position within the sample acquisition element 214. When the sample acquisition element 214 is pushed perpendicularly on the skin after lancing, the skin under the skin contact member 222 is deflected inwardly uniformly around the longer outside wall 224. By moving the skin deflection location away from the incision location, the sample acquisition element 214 does not compress the skin on opposite sides of the incision together so as to close the incision during acquisition of the fluid. As should be appreciated, by preventing premature closure of the incision, a larger quantity of fluid can be collected.

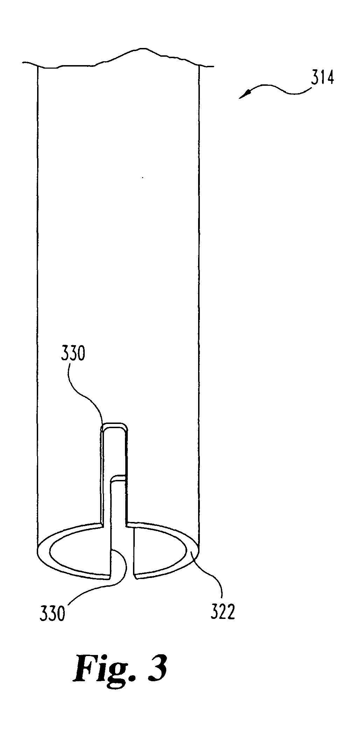

A sample acquisition element 314 according to another embodiment will now be described with reference to FIG. 3. The element 314 has a number of features that are similar to the embodiments described above with reference to FIGS. 1 and 2. For instance, the incision forming member 112 of sample acquisition element 314 is located within the sample acquisition element 314 and is movable longitudinally from a first retracted position to a second extended position. A fluid receiving space 120 is defined within the sample acquisition element 314 and the incision forming member 112. Alternatively, in another embodiment, the sample acquisition element 314 does not have an incision forming member positioned inside.

As shown in FIG. 3, the sides of the sample acquisition element 314 define one or more grooves 330 extending from skin contact surface 322. The grooves 330 are sufficiently long such that a portion of the grooves 330 is not closed by the skin when the sample acquisition element 314 is pressed against the skin during fluid acquisition. More specifically, the deflection of the skin under the skin contact member 322 does not result in the skin on either side of the sample acquisition element 314 from entirely covering the grooves 330. During fluid collection, the grooves 330 permit air to flow from outside the sample acquisition element 314 into the inside of the sample acquisition element 314, This airflow facilitates the flow of bodily fluid from the incision into the sample acquisition element 314. Moreover, body fluid in the skin can flow to the incision in the skin even when element 314 is pressed against the skin.

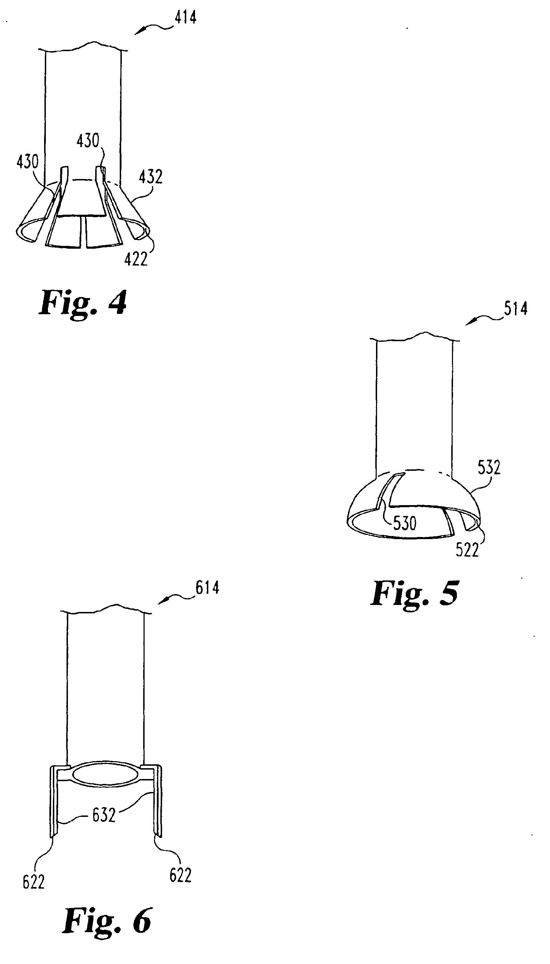

Tip designs for sample acquisition elements according to further embodiments are illustrated in FIGS. 4-6. The sample acquisition elements illustrated in FIGS. 4-6 can be used to sample fluid in a manner similar to the technique as described above with reference to FIGS. 7-9, with some of the notable differences discussed below. FIG. 4 illustrates a sample acquisition element 414 having a conically flared shaped tip 432. As a result of the conically shaped tip 432, the skin contact surface 422 is moved further away from the location of the incision. Otherwise, the skin contact surface 422 may be located too close to the incision such that when the sample acquisition element 414 is pushed perpendicularly into the skin, the skin adjacent to the incision would be pushed into the incision, thereby closing the incision. Additionally, grooves 430 through the sides of the sample acquisition element 414 extend from the skin contact surface 422 that allow fluid to flow into the incision. As discussed above, as a result of the flared tip 432 and grooves 430, the outward deflection of the skin around the incision will open the incision and the grooves 430 will facilitate the flow of blood or other bodily fluid into the sample acquisition element 414.

Although not shown in FIG. 4, the incision forming member 112 is positioned inside the sample acquisition element 414. Additionally, the incision forming member 112 is movable longitudinally from a first retracted position to a second extended position. A fluid receiving space 120 is defined within the sample acquisition element 414 and the incision forming member 112. Alternatively, in another embodiment of the sample device, the device does not have an incision forming member within the sample acquisition element 414.

A fluid sampling device 500 according to a further embodiment will now be described with reference to FIG. 5. Device 500 shares a number of features common to the one described above with reference to FIG. 4. For instance, device 500 includes a capillary member 514 with a bowl shaped flared tip 532 having grooves 530 through the sides of the capillary member 514 extending from the skin contact surface 522. Although not shown, alternatively, an incision forming member 112 may be positioned within the sample acquisition element. The bowl shaped flared tip 532 positions the skin contact surface 522 away from the incision. If the skin contact surface 522 were directly adjacent to the incision, the skin contact surface 522 would push the skin adjacent to the incision together, thus closing the incision. By positioning the skin contact surface slightly away from the incision, the skin under the capillary member 514 is stretched when the capillary member 514 is pressed perpendicularly into the skin. The inward deflection of the skin under the skin contact member 522 will tend to open, rather than close, the incision. Additionally, the grooves 530 will facilitate the flow of blood out of the incision and into the capillary member 514.

FIG. 6 illustrates a capillary member 614 of another embodiment. The capillary member 614 has flat extensions 632 protruding from the capillary member 614. The flat extensions 632 are parallel to each other and on opposite sides of the capillary member 614 and each have a skin contact surface 622 that is beveled. When the capillary member 614 is pushed perpendicularly into the skin, each flat extension 632 deflects the skin beneath the skin contact surface 622 inwardly. The inward deflection of the skin on opposite sides of the incision stretches the skin under the capillary member 614 tending to open the incision. Although not shown in FIG. 6, the incision forming member 112 is positioned inside the capillary member 614. Additionally, the incision forming member 112 is movable longitudinally from a first retracted position to a second extended position. Alternatively, in another embodiment of the sample device, the device does not have an incision forming member within the capillary member 614.

## Claims

1. A body fluid sampling device, comprising:
a sample acquisition element defining a cavity;
a incision forming member slidably disposed in the cavity to form an incision in skin, the sample acquisition element and the incision forming member defining a fluid passage that is sized to draw body fluid from the incision via capillary action; and
the sample acquisition element has a skin contact surface that is shaped to maintain the incision open as the sample acquisition element is pressed against the skin to draw the body fluid into the fluid passage, wherein the sample acquisition element comprises a first side and an opposing second side and the first side is longer than the second side.

2. A body fluid sampling device, comprising:
a sample acquisition element defining a fluid cavity sized to draw body fluid via capillary action;
a incision forming member slidably disposed in the fluid cavity to form an incision in skin; and
the sample acquisition element has a skin contact surface that is asymmetrically shaped to apply an asymmetric force to the skin surrounding the incision to keep the incision open,
wherein the sample acquisition element has a first side and an opposing second side; the first side is longer that the second side; and
the skin contact surface slopes from the first side to the second side..

3. The device of claim 2, wherein the skin contact surface is smooth.

## Patentansprüche

1. Körperflüssigkeitsentnahmevorrichtung mit
einem Probenaufnahmeelement, das einen Hohlraum definiert,
einem verschiebbar im Hohlraum angeordneten Einschnittausführungselement zum Durchführen eines Hauteinschnitts, wobei das Probenaufnahmeelement und das Einschnittausführungselement einen Flüssigkeitsdurchgang definieren, der so bemessen ist, dass Körperflüssigkeit durch Kapillarwirkung aus dem Einschnitt gesaugt wird, wobei
das Probenaufnahmeelement eine Hautkontaktfläche hat, die so geformt ist, dass der Einschnitt offen gehalten wird, während das Probenaufnahmeelement an die Haut gedrückt wird, um die Körperflüssigkeit in den Flüssigkeitsdurchgang zu saugen, wobei das Probenaufnahmeelement eine erste Seite und eine gegenüberliegende zweite Seite umfasst und die erste Seite länger als die zweite Seite ist.

2. Körperflüssigkeitsentnahmevorrichtung mit
einem Probenaufnahmeelement, das einen Flüssigkeitshohlraum definiert, der so bemessen ist, dass Körperflüssigkeit durch Kapillarwirkung angesaugt wird,
einem verschiebbar im Flüssigkeitshohlraum angeordneten Einschnittausführungselement zum Durchführen eines Hauteinschnitts, wobei das Probenaufnahmeelement eine Hautkontaktfläche hat, die asymmetrisch geformt ist, um eine asymmetrische Kraft auf die Haut um den Einschnitt herum auszuüben, um den Einschnitt offen zu halten,
wobei das Probenaufnahmeelement eine erste Seite und eine gegenüberliegende zweite Seite hat und die erste Seite länger als die zweite Seite ist und die Hautkontaktfläche von der ersten Seite zur zweiten Seite hin abfällt.

3. Vorrichtung nach Anspruch 2, wobei die Hautkontaktfläche glatt ist.

## Revendications

1. Dispositif d'échantillonnage de fluide corporel, comprenant :
un élément d'acquisition d'échantillon définissant une cavité ;
un membre formant une incision disposé en glissement dans la cavité pour former une incision dans la peau, l'élément d'acquisition d'échantillon et le membre formant une incision définissant un passage pour le fluide dimensionné pour prélever un fluide corporel à partir de l'incision via une action capillaire ; et
l'élément d'acquisition d'échantillon possédant une surface de mise en contact avec la peau qui est configurée pour maintenir l'incision ouverte lorsque l'élément d'acquisition d'échantillon est pressé contre la peau pour prélever le fluide corporel dans le passage pour le fluide, l'élément d'acquisition d'échantillon comprenant un premier côté et un deuxième côté opposé, le premier côté étant plus long que le deuxième côté.

2. Dispositif d'échantillonnage de fluide corporel, comprenant :
un élément d'acquisition d'échantillon définissant une cavité pour le fluide, dimensionnée pour prélever un fluide corporel via une action capillaire ;
un membre formant une incision disposé en glissement dans la cavité pour le fluide de façon à former une incision dans la peau ; et
l'élément d'acquisition d'échantillon possédant une surface de mise en contact avec la peau qui est de configuration asymétrique pour exercer une force asymétrique sur la peau entourant l'incision afin de maintenir l'incision ouverte ;
dans lequel l'élément d'acquisition d'échantillon possède un premier côté et un deuxième côté opposé, le premier côté étant plus long que le deuxième côté ; et
la surface de mise en contact avec la peau étant inclinée dans la direction allant du premier côté au deuxième côté.

3. Dispositif selon la revendication 2, dans lequel la surface de mise en contact avec la peau est une surface lisse.
